(19) Europäisches Patentamt
European Patent Office
Office européen des brevets

(11) Veröffentlichungsnummer: **0 282 864 B1**

(12) **EUROPÄISCHE PATENTSCHRIFT**

(45) Veröffentlichungstag der Patentschrift: **04.08.93**

(51) Int. Cl.5: **A61K 7/48**, A61K 7/06, B01F 17/18

(21) Anmeldenummer: **88103508.3**

(22) Anmeldetag: **07.03.88**

(54) **Pumpfähige kationische Fettalkoholdispersion.**

(30) Priorität: **16.03.87 DE 3708425**

(43) Veröffentlichungstag der Anmeldung:
**21.09.88 Patentblatt 88/38**

(45) Bekanntmachung des Hinweises auf die Patenterteilung:
**04.08.93 Patentblatt 93/31**

(84) Benannte Vertragsstaaten:
**AT BE CH DE ES FR GB GR IT LI NL SE**

(56) Entgegenhaltungen:
**EP-A- 0 155 806**
**EP-A- 0 181 547**
**WO-A-86/02828**

**PATENT ABSTRACTS OF JAPAN,Band 12, Nr 132 (C490)(2979),22. april 1988; 1988; JP -A- 62 252 710 (KAO CORPORATION) 04.11.1987 (Kat. x,p)* Zusammenfassung***

**PATENT ABSTRACTS OF JAPAN, Band 7 Nr 181 ( c-180) (1326),10. August 1985; & JP A 58 85810 (Kao Sekken K.K ) 23.05.1983 * Zusammenfassung*.**

(73) Patentinhaber: **Henkel Kommanditgesellschaft auf Aktien**

**W-4000 Düsseldorf-Holthausen(DE)**

(72) Erfinder: **Kawa, Rolf**
**Humboldtstrasse 47**
**W-4019 Monheim(DE)**
Erfinder: **Tesmann, Holger, Dr.**
**Vennstrasse 47**
**W-4000 Düsseldorf 12(DE)**
Erfinder: **Wilhelm, Josef**
**Am Mühlgraben 4**
**W-6418 Hünfeld(DE)**
Erfinder: **Rose, Karl-Heinz**
**Athanasius-Kircher-Strasse 10**
**W-6418 Hünfeld(DE)**
Erfinder: **Konrad, Eugen**
**Mecklenburger Strasse 101**
**W-6100 Darmstadt(DE)**

**EP 0 282 864 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine pumpfähige kationische Fettalkoholdispersion mit einem hohen Gehalt an dispergiertem Fettalkohol und einem geringem Gehalt an kationischem Dispergator.

Für die Herstellung zahlreicher flüssiger, wäßriger und emulsionsförmiger Zubereitungen zur Pflege der Haut und der Haare werden Fettalkohole verwendet, die in dispergierter Form in diesen Zubereitungen der Haut eine glatte und geschmeidige Oberfläche und dem Haar eine verbesserte Kämmbarkeit und Avivage verleihen. Die Einarbeitung der Fettalkohole, die bei Normaltemperatur feste, wachsartige Stoffe sind, erfordert ein Aufschmelzen und eine gleichmäßige stabile Dispergierung in der ebenfalls erwärmten Lösung oder Emulsion der übrigen Komponenten.

So sind z. B. aus EP-0 181 547 A2 haarkosmetische Zusammensetzungen bekannt, die bis zu 4 Gew.-% Cetylalkohol enthalten, der gemeinsam mit verzweigtkettigen quartären Ammoniumverbindungen in Form einer Schmelze der wässrigen Phase zugesetzt wird.

Diese Arbeitsweise ist mit erheblichem technischen Aufwand verbunden, so daß es für viele Hersteller von Kosmetika und kleinere Mischbetriebe von großem Vorteil wäre, wenn der Fettalkohol bereits in Form eines stabilen Dispersionskonzentrats verfügbar wäre, welches den Präparaten ohne Erwärmen und ohne spezielle Dispergiereinrichtungen zugesetzt und durch bloßes Rühren homogen darin verteilt werden kann. Diesem Bedürfnis wird durch die vorliegende Erfindung abgeholfen.

Gegenstand der Erfindung ist eine pumpfähige kationische Fettalkoholdispersion mit einem hohen Gehalt an dispergiertem Fettalkohol und einem Gehalt an kationischem Dispergator, dadurch gekennzeichnet, daß sie

(A) 10,1 bis 25,0 Gew.-% eines linearen, oder verzweigten primären, gesättigten Fettalkohols mit 14 bis 22 C-Atomen,

(B) 0,01 bis 1 Gew.-% einer kationischen oberflächenaktiven Verbindung mit einer quartären Ammonium-, Pyridinium- oder Imidazoliniumgruppe und einer linearen Alkyl- oder 2-Hydroxyalkylgruppe mit 8 bis 22 C-Atomen und

(C) 70,0 bis 89 Gew.-% Wasser

enthält.

Es wurde überraschend festgestellt, daß die erfindungsgemäßen Fettalkoholdispersionen mit sehr geringen Mengen der kationischen oberflächenaktiven Verbindung stabilisiert werden und daß insbesondere bei niedrigen Gehalten an kationischem Dispergator trotz hoher Fettalkoholgehalte eine niedrige Viskosität und damit eine gute Pumpbarkeit der Dispersion erreicht wird.

Bevorzugt enthalten die erfindungsgemäßen Fettalkoholdispersionen daher

(A) 16,0 bis 25,0 Gew.-% des Fettalkohols,

(B) 0,01 bis 0,5-Gew.-% der kationischen oberflächenaktiven Verbindung und

(C) 74,0 bis 83,0 Gew.-% Wasser.

Die linearen, primären, gesättigten Fettalkohole wie z. B. Myristylalkohol, Cetylalkohol, Stearylalkohol, Arachidylalkohol oder Behenylalkohol können einzeln oder als technische Gemische enthalten sein. Bevorzugt eignen sich Cetyl- und/oder Stearylalkohol. Diese Alkohole sind auch als Cetyl-Stearylalkohol-Schnitte mit unterschiedlichen Anteilen an Cetylalkohol und Stearylalkohol im Handel.

Als kationische oberflächenaktive Verbindungen mit einer für den Erfindungszweck erforderlichen Dispergierwirkung eignen sich Verbindungen, die neben einer wasserlöslich machenden, quartären Ammonium-, Pyridinium- oder Imidazoliniumgruppe eine lipophile, lineare Alkyl- oder 2-Hydroxyalkylgruppe mit 8 bis 22 C-Atomen enthalten. Solche Verbindungen sind z. B. Cetylpyridiniumchlorid, 2-Alkyl($C_{15}$)-1-(2'-hydroxyethyl)-1-methylimidazolinium-methosulfat, 2-Undecyl-1-(2-hydroxyethyl)-1-methyl-imidazoliniumchlorid oder 2-Talgalkyl-1-Talgacylaminoethyl-ethyl-imidazoliniumethosulfat.

Unter den zahlreichen quartären Ammoniumverbindungen kommt im Hinblick auf eine besonders gute Dispergierwirkung den Verbindungen der Formel (I)

(I) 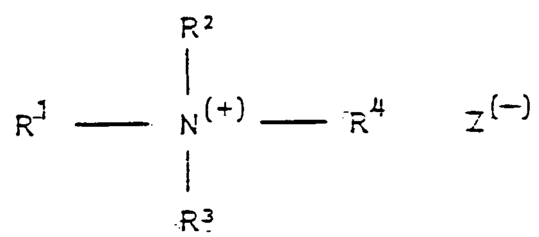

in der $R^1$ eine Alkyl- oder 2-Hydroxyalkylgruppe mit 8 bis 22 C-Atomen, eine Gruppe $R^5CONH(CH_2)_x$-, worin $R^5$ eine Alkylgruppe mit 7 bis 21 C-Atomen und x eine ganze Zahl von 2 bis 4 ist, $R^2$ und $R^3$ eine Alkylgruppe mit 1 bis 4 C-Atomen oder eine Gruppe der Formel $-(C_nH_{2n}O)_y-H$ ist, worin n eine ganze Zahl von 2 bis 4 und y eine Zahl von 1 bis 10 ist, und $R^4$ eine Benzylgruppe ist oder eine der für $R^2$ und $R^3$ angegebenen Bedeutungen hat und $Z^{(-)}$ ein Chlorid, Bromid, Hydrogensulfat, Hydrogenphosphat, Methoxysulfat oder Ethoxysulfat-Anion ist, eine besondere Bedeutung für die vorliegende Erfindung zu. Besonders geeignete Beispiele solcher kationischer oberflächenaktiver Verbindungen sind z. B. Cetyl-trimethylammoniumchlorid, Lauryl-dimethyl-benzyl-ammoniumchlorid, 2-Hydroxycetyl-2-hydroxyethyl-dimethyl-ammoniumchlorid, Lauryl-trimethylammoniumchlorid oder Behenyl-trimethylammoniumchlorid.

Unter den kationischen oberflächenaktiven quartären Ammoniumverbindungen der Formel (I) sind besonders solche bevorzugt als Dispergiermittel für die erfindungsgemäße Fettalkoholdispersion geeignet, in welchen $R^1$ eine Alkyl- oder 2-Hydroxyalkylgruppe mit 12 bis 18 C-Atomen, $R^2$, $R^3$ und $R^4$ Methyl- oder 2-Hydroxyethylgruppen und $Z^{(-)}$ ein Chloridanion ist.

Darüber hinaus können die erfindungsgemäßen Fettalkoholdispersionen noch in untergeordneten Mengen bis etwa 5 Gew.-% weitere Hilfs- und Zusatzmittel enthalten, wie z. B. Paraffine, Glycerinmonostearat, Ethylenglykoldistearat, Fettsäurealkanolamide, Duftstoffe, Farbstoffe, Konservierungsstoffe, Komplexbildner oder Salze und Puffersubstanzen.

Der pH-Wert der erfindungsgemäßen Fettalkoholdispersion liegt bevorzugt in einem Bereich zwischen pH = 2 und pH = 10. Zur Einstellung können hierfür geeignete Puffersubstanzen wie z. B. Citronensäure oder Triethanolamin in einer Menge von 0,1 bis 1 Gew.-% eingesetzt werden.

Die Herstellung der erfindungsgemäßen Fettalkoholdispersion kann in einfacher Weise so durchgeführt werden, daß der Fettalkohol (A) über seinen Schmelzpunkt erwärmt wird und die Schmelze bei einer Temperatur von bis zu 50 °C oberhalb des Schmelzpunktes mit der ebenfalls über die Schmelztemperatur des Fettalkohols erwärmten Lösung der kationischen oberflächenaktiven Verbindung (B) in Wasser (C) in einer statischen oder dynamischen Misch-oder Dispergiereinrichtung gemischt und homogenisiert wird. Eine besonders bewährte Verfahrensweise zeichnet sich dadurch aus, daß man den Fettakohol (A) und die wäßrige Lösung der kationischen oberflächenaktiven Verbindung (B) auf 70 bis 80 °C erwärmt und in einem Rotor-Stator-Homogenisator vereinigt und die sich bildende Dispersion unter ständiger Bewegung, z. B. unter Rühren, auf eine Temperatur unterhalb des Schmelzpunktes des Fettalkohols abkühlt.

Die erfindungsgemäße Fettalkoholdispersion ist von fließfähiger Konsistenz und daher auch bei Normaltemperatur von 20 °C problemlos pumpfähig. Ihre Viskosität liegt im allgemeinen in einem Bereich von 0,5 bis 10 Pa.s (20 °C, bei Messung mit einem Brookfield-Rotationsviskosimeter) und kann in ungünstigen Fällen bis ca. 50 Pa.s (20 °C) ansteigen, z. B. bei hohen Konzentrationen von über 20 Gew.-% Fettalkohol und mehr als 0,2 Gew.-% der kationischen oberflächenaktiven Verbindung und längerer Lagerzeit. Es bleibt aber stets eine gute Pumpfähigkeit und eine leichte, spontane Verdünnbarkeit mit Wasser oder eine spontane Verteilbarkeit in wäßrigen Zubereitungen erhalten. Die erfindungsgemäße Fettalkoholdispersion stellt daher eine besonders günstige, leicht verarbeitbare Lieferform für Fettalkohole dar.

Sie eignet sich insbesondere zur Herstellung wäßriger kosmetischer Zubereitungen zur Pflege der Haut und der Haare, die Fettalkohole mit 14 bis 22 C-Atomen in fein dispergierter Form enthalten. Eine besonders bevorzugte Verwendung finden die erfindungsgemäßen Fettalkoholdispersionen zur Herstellung von Haarnachbehandlungsmitteln bzw. Haarspülmitteln, die nach der Haarwäsche auf das Kopfhaar aufgebracht werden, um die Kämmbarkeit zu verbessern, die statische Aufladbarkeit zu verringern und dem Haar Glanz, Halt und Fülle zu verleihen. Typische Haarnachspülmittel enthalten für diesen Zweck 0,5 bis 5 Gew.-% Fettalkohol in fein dispergierter Form sowie 0,3 bis 3 Gew.-% kationischer oberflächenaktiver Verbindungen. Solche Haarspülmittel lassen sich aus der erfindungsgemäßen Fettalkoholdispersion durch entsprechende Verdünnung mit einer wäßrigen Lösung einer kationischen oberflächenaktiven Verbindung ohne besondere Homogenisier- oder Dispergiereinrichtungen bei Normaltemperatur herstellen.

Die folgenden Beispiele sollen den Gegenstand der Erfindung näher erläutern.

## Beispiele

Die in den Tabellen I und II aufgeführten Fettalkoholdispersionen wurden wie folgt hergestellt:

Eine Mischung aus Wasser, quartärer Ammoniumverbindung wurde auf 75 °C erwärmt und mit der auf 75 °C erwärmten Schmelze der Fettalkohole in einem Homogenisator vom Rotor-Stator-Typ unter Einsatz hoher Scherkräfte dispergiert. Dann wurde die Dispersion unter stetigem Rühren auf 40 °C abgekühlt.

Die in den Tabellen I und II beschriebenen Fettalkoholdispersionen blieben über 4 Wochen unverändert stabil. Innerhalb der ersten Woche erfolgte meist ein leichter Viskositätsanstieg.

Tabelle I

| | 1 | 2 | 3 | 4 | 5 | 6 | 7 | 8 | 9 |
|---|---|---|---|---|---|---|---|---|---|
| Cetyl-/Stearylalkohol (1:1) | 15 | 15 | 15 | 20 | 20 | 20 | 25 | 25 | 25 |
| Cetyltrimethylammoniumchlorid | 0,025 | 0,05 | 0,1 | 0,025 | 0,05 | 0,1 | 0,025 | 0,1 | 0,2 |
| Wasser | ad | ad | ad | ad | ad | ad | ad | ad | ad |
| | 100 | 100 | 100 | 100 | 100 | 100 | 100 | 100 | 100 |
| Viskosität, Brookfield-Rotationsviskosimeter, Typ RVF, Spindel No. 5, 10 UpM, 20 °C | | | | | | | | | |
| nach 24 Stunden (Pa.s) | 1,6 | 3,0 | 7,2 | 1,8 | 4,0 | 9,2 | 4,8 | 11,6 | 19,2 |
| nach 7 Tagen (Pa.s) | 2,4 | 4,4 | 9,2 | 2,4 | 5,0 | 11,2 | 4,8 | 13,2 | 28,0 |

Tabelle II

| | 10 | 11 | 12 | 13 | 14 | 15 |
|---|---|---|---|---|---|---|
| Myristylalkohol | 20 | 20 | 20 | - | - | - |
| Behenylalkohol | - | - | - | 20 | 20 | 20 |
| Cetyltrimethylammoniumchlorid | 0,1 | - | - | 0,1 | - | - |
| 2-Hydroxyethyl-2-hydroxyethyldimethylammoniumchlorid | - | 0,1 | - | - | 0,1 | - |
| Behenyltrimethylammoniumchlorid | - | - | 0,1 | - | - | 0,1 |
| Wasser | ad | ad | ad | ad | ad | ad |
| | 100 | 100 | 100 | 100 | 100 | 100 |
| Viskosität, Brookfield-Rotationsviskosimeter, Typ RVF, Spindel No. 5, 10 UpM, 20 °C | | | | | | |
| nach 24 Stunden (Pa.s) | 3 | 4,4 | 8,2 | 14,4 | 11,2 | 4,8 |
| nach 7 Tagen (Pa.s) | 11 | 6,5 | 15,2 | 12,4 | 12,8 | 6,8 |

## Patentansprüche

**1.** Pumpfähige kationische Fettalkoholdispersion, dadurch gekennzeichnet, daß sie

(A) 10,1 bis 25,0 Gew.-% eines linearen oder verzweigten primären, gesättigten Fettalkohols mit 14 bis 22 C-Atomen,

(B) 0,01 bis 1,0 Gew.-% einer kationischen oberflächenaktiven Verbindung mit einer quartären Ammonium-, Pyridinium- oder Imidazoliniumgruppe und einer linearen Alkyl- oder 2-Hydroxyalkylgruppe mit 8 bis 22 C-Atomen und

(C) 70,0 bis 89 Gew.-% Wasser

enthält.

**2.** Fettakoholdispersion nach Anspruch 1, dadurch gekennzeichnet, daß sie

(A) 16,0 bis 25,0 Gew.-% des Fettalkohols,

(B) 0,01 bis 0,5 Gew.-% der kationischen oberflächenaktiven Verbindung und

(C) 74,0 bis 83,0 Gew.-% Wasser

enthält.

**3.** Fettalkoholdispersion nach den Ansprüchen 1 oder 2, dadurch gekennzeichnet, daß der Fettalkohol (A) ein Cetyl- und/oder Stearylalkohol ist.

**4.** Fettalkoholdispersion nach den Ansprüchen 1 bis 3, dadurch gekennzeichnet, daß die kationische, oberflächenaktive Verbindung (B) eine quartäre Ammoniumverbindung der Formel (I)

(I) 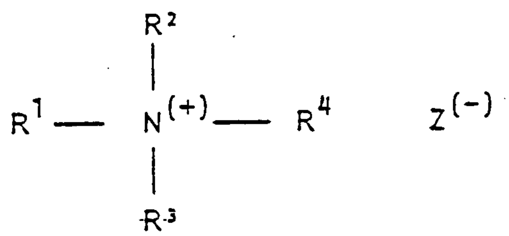

ist, in der $R^1$ eine Alkyl- oder 2-Hydroxyalkylgruppe mit 8 bis 22 C-Atomen, eine Gruppe $R^5CONH-(CH_2)_x-$, worin $R^5$ eine Alkylgruppe mit 7 bis 21 C-Atomen und x eine ganze Zahl von 2 bis 4 ist, $R^2$ und $R^3$ eine Alkylgruppe mit 1 bis 4 C-Atomen oder eine Gruppe der Formel $-(C_nH_{2n}O)_yH$, worin n eine ganze Zahl von 2 bis 4 und y eine Zahl von 1 bis 10 ist, und $R^4$ eine Benzylgruppe ist oder eine der für $R^2$ und $R^3$ angegebenen Bedeutungen hat, und $Z^{(-)}$ ein Chlorid-, Bromid-, Hydrogensulfat-, Hydrogenphosphat-, Methoxysulfat oder Ethoxysulfat-Anion ist.

**5.** Fettalkoholdispersion nach den Ansprüchen 1 bis 4, dadurch gekennzeichnet, daß zusätzlich 0,1 bis 1 Gew.-% an Puffersubstanzen zur Einstellung eines pH-Wertes der Dispersion von 2 bis 10 enthalten sind.

**6.** Fettalkoholdispersion nach den Ansprüche 1 bis 5, dadurch gekennzeichnet, daß als kationische oberflächenaktive Verbindung (B) eine quartäre Ammoniumverbindung der Formel (I) gemäß Anspruch 4 enthalten ist, in der $R^1$ eine Alkyl-oder 2-Hydroxyalkylgruppe mit 12 bis 18 C-Atomen, $R^2$, $R^3$ und $R^4$ Methyl- oder 2-Hydroxyethylgruppen sind und $Z^{(-)}$ ein Chloridanion ist.

**7.** Verfahren zur Herstellung einer Fettalkoholdispersion gemäß den Ansprüchen 1 bis 6, dadurch gekennzeichnet, daß der Fettalkohol (A) über seinen Schmelzpunkt erwärmt wird und die Schmelze bei einer Temperatur von bis zu 50 °C oberhalb des Schmelzpunktes mit der ebenfalls über die Schmelztemperatur des Fettalkohols erwärmten Lösung der kationischen oberflächenaktiven Verbindung (B) in Wasser (C) in einer statischen oder dynamischen Misch- oder Dispergiereinrichtung gemischt und homogenisiert wird.

**8.** Verfahren nach Anspruch 7, dadurch gekennzeichnet, daß der Fettalkohol (A) und die wäßrige Lösung der kationischen oberflächenaktiven Verbindung (B) auf 70 bis 80 °C erwärmt und in einem Rotor-Stator-Homogenisator vereinigt werden und die sich bildende Dispersion unter ständiger Bewegung auf eine Temperatur unterhalb des Schmelzpunktes des Fettalkohols abgekühlt wird.

**9.** Verwendung einer Fettalkoholdispersion gemäß den Ansprüchen 1 bis 6 zur Herstellung von wäßrigen, kosmetischen Zubereitungen zur Pflege der Haut und der Haare.

## Claims

**1.** A pumpable cationic fatty alcohol dispersion, characterized in that it contains
(A) 10.1 to 25.0% by weight of a linear or branched, primary, saturated $C_{14-22}$ fatty alcohol,
(B) 0.01 to 1.0% by weight of a cationic surface-active compound containing a quaternary ammonium, pyridinium or imidazolinium group and a linear $C_{8-22}$ alkyl or 2-hydroxyalkyl group and
(C) 70.0 to 89 % by weight water.

**2.** A fatty alcohol dispersion as claimed in claim 1, characterized in that it contains
(A) 16.0 to 25.0% by weight of the fatty alcohol,
(B) 0.01 to 0.5% by weight of the cationic surface-active compound and
(C) 74.0 to 83.0% by weight water.

**3.** A fatty alcohol dispersion as claimed in claim 1 or 2, characterized in that the fatty alcohol (A) is a cetyl and/or stearyl alcohol.

**4.** A fatty alcohol dispersion as claimed in claims 1 to 3, characterized in that the cationic surface-active compound (B) is a quaternary ammonium compound corresponding to the following formula

(I) 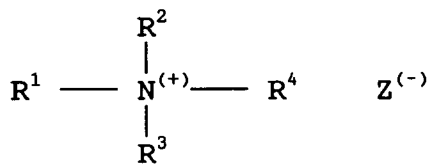

in which $R^1$ is a $C_{8-22}$ alkyl or 2-hydroxyalkyl group, a group of the formula $R^5CONH(CH_2)_x$-, where $R^5$ is a $C_{7-21}$ alkyl group and x is an integer of 2 to 4, $R^2$ and $R^3$ represent a $C_{1-4}$ alkyl group or a group of the formula $-(C_nH_{2n}O)_y-H$, where n is an integer of 2 to 4 and y is a number of 1 to 10, and $R^4$ is a benzyl group or has one of the meanings defined for $R^2$ and $R^3$ and $Z^{(-)}$ is a chloride, bromide, hydrogen sulfate, hydrogen phosphate, methoxysulfate or ethoxysulfate anion.

**5.** A fatty alcohol dispersion as claimed in claims 1 to 4, characterized in that it additionally contains from 0.1 to 1% by weight of buffer substances to adjust the dispersion to a pH value of from 2 to 10.

**6.** A fatty alcohol dispersion as claimed in claims 1 to 5, characterized in that the cationic surface-active compound (B) is a quaternary ammonium compound corresponding to formula (I) in claim 4, in which $R^1$ is a $C_{12-18}$ alkyl or 2-hydroxyalkyl group, $R^2$, $R^3$ and $R^4$ are methyl or 2-hydroxyethyl groups and $Z^{(-)}$ is a chloride anion.

**7.** A process for the production of the fatty alcohol dispersion claimed in claims 1 to 6, characterized in that the fatty alcohol (A) is heated beyond its melting point and the resulting melt is mixed and homogenized at a temperature of up to 50 °C above the melting point with a solution of the cationic surface-active compound (B) in water (C) similarly heated beyond the melting point of the fatty alcohol in a static or dynamic mixer or dispersion unit.

**8.** A process as claimed in claim 7, characterized in that the fatty alcohol (A) and the aqueous solution of the cationic surface-active compound (B) are heated to 70 to 80 °C and combined in a rotor-stator homogenizer and the dispersion formed is cooled with continuous agitation to a temperature below the melting point of the fatty alcohol.

**9.** The use of the fatty alcohol dispersion claimed in claims 1 to 6 for the production of aqueous, cosmetic hair-care and skin-care preparations.

**Revendications**

**1.** Dispersion cationique pompable d'alcool gras, caractérisée en ce qu'elle renferme
(A) 10,1 à 25,0 % en poids d'un alcool gras saturé, primaire, linéaire ou ramifié comportant 14 à 22 atomes de C,
(B) 0,01 à 1,0 % en poids d'un composé tensioactif cationique comportant un groupe d'ammonium, pyridinium ou imidazolinium quaterneire et un groupe alkyle ou 2-hydroxyalkyle linéaire, comprenant 8 à 22 atomes de C et
(C) 70,0 à 89 % en poids d'eau.

**2.** Dispersion d'alcool gras selon la revendication 1, caractérisée en ce qu'elle renferme
(A) 16,0 à 25,0 % en poids de l'alcool gras,
(B) 0,01 à 0,5 % en poids du composé tensioactif cationique et
(C) 76,0 à 83,0 % en poids d'eau.

**3.** Dispersion d'alcool gras selon les revendications 1 ou 2, caractérisée en ce que l'alcool gras (A) est un alcool cétylique et/ou stéarylique.

**4.** Dispersion d'alcool gras selon les revendications 1 à 3, caractérisée en ce que le composé tensioactif cationique (B) est un composé d'ammonium quaternaire de la formule (I)

(I) 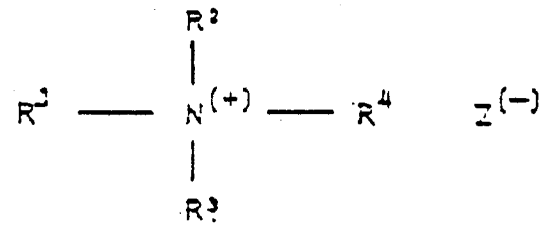

dans laquelle $R^1$ représente un groupe alkyle ou 2-hydroxyalkyle comportant 8 à 22 atomes de C, un groupe $R^5CONH(CH_2)_x$-, dans lequel $R^5$ correspond à un groupe alkyle comprenant 7 à 21 atomes de C et où x est un nombre entier allant de 2 à 4, $R^2$ et $R^3$ représentent un groupe alkyle comportant 1 à 4 atomes de C ou un groupe de la formule - $(C_nH_{2n}O)_yH$, dans laquelle n correspond à un nombre entier allant de 2 à 4 et où y est un nombre allant de 1 à 10, $R^4$ représente un groupe benzyle ou possède une des significations apécifiées pour $R^2$ et $R^3$, et $Z^{(-)}$ représente un anion chlorure, bromure, hydrogénosulfate, hydrogénophosphate, méthoxysulfate ou éthosysulfate.

**5.** Dispersion d'alcool gras selon les revendications 1 à 4, caractérisée en ce qu'elle renferme en plus 0,1 à 1 % en poids de substances tampons pour ajuster le pH de la dispersion entre 2 et 10.

**6.** Dispersion d'alcool gras selon les revendications 1 à 5, caractérisée en ce qu'elle renferme, comme composé tensioactif cationique (B), un composé d'ammonium quaternaire de la formule (I), selon la revendication 4, dans laquelle $R^1$ représente un groupe alkyle ou 2-hydroxyalkyle comportant 12 à 18 atomes de C, $R^2$, $R^3$ et $R^4$ correspondent à des groupes méthyle ou 2-hydroxyéthyle, et $Z^{(-)}$ est un anion chlorure.

**7.** Procédé de fabrication d'une dispersion d'alcool gras selon les revendications 1 à 6, caractérisé en ce que l'alcool gras (A) est chauffé au-delà de son point de fusion et en ce que la masse fondue est mélangée et homogénéisée dans un dispositif de mélange ou de dispersion statique ou dynamique, à une température allant jusqu'à 50 °C au-delà du point de fusion, avec la solution du composé tensioactif cationique (B) dans l'eau (C), également chauffée au-delà de la température de fusion de l'alcool gras.

**8.** Procédé selon la revendication 7, caractérisé en ce que l'alcool gras (A) et la solution aqueuse du composé tensioactif cationique (B) sont chauffée à 70-80 °C et réunis dans un homogénéisateur à rotor-stator, et en ce que la dispersion se formant est refroidie sous agitation constante à une température inférieure au point de fusion de l'alcool gras.

**9.** Mise en oeuvre d'une dispersion d'alcool gras selon les revendications 1 à 6 pour la fabrication de préparations cosmétiques aqueuses pour soigner la peau et les cheveux.